# EUROPEAN PATENT SPECIFICATION

(11) **EP 4 061 236 B1**
(45) Date of publication and mention of the grant of the patent: **01.05.2024**
(21) Application number: 20829455.3
(22) Date of filing: 04.12.2020
(51) Int. Cl.: A61B 8/08, A61B 5/06, A61B 17/34, A61M 5/32

(54) **NEEDLE-GUIDANCE SYSTEMS, COMPONENTS, AND METHODS THEREOF**
NADELFÜHRUNGSSYSTEME, KOMPONENTEN UND VERFAHREN DAFÜR
SYSTÈMES DE GUIDAGE D'AIGUILLE, ÉLÉMENTS ET PROCÉDÉS ASSOCIÉS

(30) Priority: 04.12.2019 US 201962943574 P
(43) Date of publication of application: 28.09.2022
(73) Proprietor: Bard Access Systems, Inc., Salt Lake City, UT 84116 (US)
(72) Inventor: PRINCE, Matthew J., Herriman, Utah 84096 (US); ROBBINS, Tab, Layton, Utah 84041 (US); NEWBY, Mark, Kamas, Utah 84036 (US); WILKINSON, Bradley M., Haledon, New Jersey 07508 (US); PETERSON, Bart, Farmington, Utah 84025 (US)
(74) Representative: Hoffmann Eitle
(86) International application number: PCT/US2020/063441
(87) International publication number: WO 2021/113733

(56) References cited:
- US-A1- 2017 079 551
- US-A1- 2018 061 546
- US-A1- 2018 289 929
- US-A1- 2018 310 955

## Description

### BACKGROUND

Existing needle-guidance systems rely on magnetized needles for guiding needle insertions into blood vessels. Disposable needle magnetizers provided with stock needles are used to produce the magnetized needles on demand, but such needle magnetizers increase both operating costs and landfill waste.

Existing needle-guidance systems also rely on proper needle selection before guiding any particular needle into a blood vessel. Needle selection in such needle-guidance systems is often automated upon reading in needle-related information by disposable radiofrequency identification ("RFID")-tag readers provided with the stock needles; however, such RFID-tag readers also increase both operating costs and landfill waste.

There is an ongoing need to reduce operating costs for both medical facilities and patients alike. There is also an ongoing need to reduce landfill waste for the sake of the environment. Disclosed herein are needle-guidance systems, components, and methods that address the foregoing needs.
US 2018/310955 A1 relates to a magnetizing system for use in selectively magnetizing a needle of a medical device.

### SUMMARY

Disclosed herein is a needle-guidance system including, in some embodiments, a console, a needle magnetizer, and an ultrasound probe. The console includes memory and a processor configured to instantiate a needle-guidance process for guiding insertion of a needle into a blood vessel of a patient. The needle-guidance process uses a combination of ultrasound-imaging data and magnetic-field data received by the console for guiding the insertion of the needle into the blood vessel of the patient. The needle magnetizer is incorporated into the console. The needle magnetizer is configured to magnetize the needle. The ultrasound probe is configured to provide to the console electrical signals corresponding to both the ultrasound-imaging data and the magnetic-field data. The ultrasound probe includes an array of piezoelectric transducers and an array of magnetic sensors. The array of piezoelectric transducers is configured to convert reflected ultrasound signals from the patient into an ultrasound-imaging portion of the electrical signals. The array of magnetic sensors is configured to convert magnetic signals from the magnetized needle into a magnetic-field portion of the electrical signals.

In some embodiments, the needle magnetizer is removably coupled to a side, top, or back of the console.

In some embodiments, the needle magnetizer is irremovably coupled to a side, top, or back of the console.

In some embodiments, the needle magnetizer includes a single permanent magnet or a plurality of permanent magnets disposed within a body of the needle magnetizer for magnetizing the needle.

In some embodiments, the permanent magnets are disposed within the body of the needle magnetizer in a multipole arrangement.

In some embodiments, the permanent magnets are annular magnets disposed within the body of the needle magnetizer in a stacked arrangement.

In some embodiments, the permanent magnet is hollow cylindrical magnet disposed within the body of the needle magnetizer.

In some embodiments, the needle magnetizer includes a single electromagnet or a plurality of electromagnets disposed within a body of the needle magnetizer for magnetizing the needle.

In some embodiments, the needle-guidance system further includes an RFID-tag reader incorporated into the console. The RFID-tag reader is configured to emit interrogating radio waves into a passive RFID tag for the needle and read electronically stored information from the RFID tag.

In some embodiments, the needle-guidance process is configured to adjust needle-guidance parameters in accordance with the electronically stored information read from the RFID tag.

In some embodiments, the needle-guidance system further includes a display screen configured for graphically guiding the insertion of the needle into the blood vessel of the patient.

Also disclosed herein is a console for a needle-guidance system including, in some embodiments, memory and a processor, a needle magnetizer, a probe interface, and a display screen. The console is configured to instantiate a needle-guidance process in the memory for guiding insertion of a needle into a blood vessel of a patient. The needle-guidance process uses a combination of ultrasound-imaging data and magnetic-field data received by the console for guiding the insertion of the needle into the blood vessel of the patient. The needle magnetizer is incorporated into the console. The needle magnetizer is configured to magnetize the needle. The probe interface is configured to provide to the console electrical signals from an ultrasound probe. The electrical signals correspond to both the ultrasound-imaging data and the magnetic-field data. The display screen is configured for graphically guiding the insertion of the needle into the blood vessel of the patient.

In some embodiments, the needle magnetizer is removably coupled to a side, top, or back of the console.

In some embodiments, the needle magnetizer is irremovably coupled to a side, top, or back of the console.

In some embodiments, the needle magnetizer includes a single permanent magnet or a plurality of permanent magnets disposed within a body of the needle magnetizer for magnetizing the needle.

In some embodiments, the needle magnetizer includes a single electromagnet or a plurality of electromagnets disposed within a body of the needle magnetizer for magnetizing the needle.

In some embodiments, the console further includes an RFID-tag reader incorporated into the console. The RFID-tag reader configured to emit interrogating radio waves into a passive RFID tag for the needle and read electronically stored information from the RFID tag.

In some embodiments, the needle-guidance process is configured to adjust needle-guidance parameters in accordance with the electronically stored information read from the RFID tag.

Also disclosed herein is a method of a needle-guidance system including, in some embodiments, an instantiating step of instantiating in memory of a console a needle-guidance process for guiding insertion of a needle into a blood vessel of a patient using a combination of ultrasound-imaging data and magnetic-field data. The method further includes a magnetizing step of magnetizing the needle with a needle magnetizer incorporated into the console when the needle is inserted into the needle magnetizer. The magnetizing step produces a magnetized needle. The method further includes a loading step of loading the ultrasound-imaging data and the magnetic-field data in the memory. The ultrasound-imaging data and magnetic-field data correspond to electrical signals received from an ultrasound probe. The method further includes a processing step of processing the ultrasound-imaging data and the magnetic-field data with a processor of the console. The method further includes a guiding step of graphically guiding the insertion of the magnetized needle into the blood vessel of the patient on a display screen of the console.

In some embodiments, the method further includes a reading step of reading into the memory electronically stored information from a passive RFID tag for the needle. The reading step is affected with interrogating radio waves emitted by an RFID-tag reader incorporated into the console.

In some embodiments, the method further includes an adjusting step of adjusting needle-guidance parameters in the needle-guidance process in accordance with the electronically stored information read from the RFID tag.

In some embodiments, the method further includes an ultrasound-signal converting step of converting patient-reflected ultrasound signals into an ultrasound-imaging portion of the electrical signals with an array of piezoelectric transducers of the ultrasound probe.

In some embodiments, the method further includes a magnetic-signal converting step of converting magnetic signals into a magnetic-field portion of the electrical signals with an array of magnetic sensors of the ultrasound probe.

Also disclosed herein is a method for a needle-guidance system including, in some embodiments, an obtaining step of obtaining a needle. The method further includes an inserting step of inserting the needle into a needle magnetizer incorporated into a console to produce a magnetized needle. The method further includes an imaging step of imaging a blood vessel of a patient with an ultrasound probe to produce ultrasound-imaging data. The method further includes an orienting step of orienting the magnetized needle for insertion into the blood vessel of the patient to produce magnetic-field data while imaging the blood vessel with the ultrasound probe. The method further includes an inserting step of inserting the magnetized needle into the blood vessel of the patient in accordance with graphical guidance on a display screen of the console for insertion of the magnetized needle into the blood vessel of the patient. The guidance is provided by a needle-guidance process instantiated by the console upon processing a combination of the ultrasound-imaging data and the magnetic-field data.

In some embodiments, the method further includes a causing step of causing an RFID-tag reader incorporated into the console to read electronically stored information from a passive RFID tag for the needle before producing the magnetized needle.

In some embodiments, the causing step further causes the needle-guidance process to adjust needle-guidance parameters for the insertion of the magnetized needle into the blood vessel of the patient in accordance with the electronically stored information read from the RFID tag.

These and other features of the concepts provided herein will become more apparent to those of skill in the art in view of the accompanying drawings and following description, which describe particular embodiments of such concepts in greater detail.

### DRAWINGS

FIG. 1 illustrates a needle-guidance system and a patient in accordance with some embodiments.
FIG. 2 illustrates a back of a console of a needle-guidance system in accordance with some embodiments.
FIG. 3 illustrates a front of the console of FIG. 2 in accordance with some embodiments.
FIG. 4 illustrates another console of the needle-guidance system in accordance with some embodiments.
FIG. 5 illustrates another console of the needle-guidance system in accordance with some embodiments.
FIG. 6 illustrates another console of the needle-guidance system in accordance with some embodiments.
FIG. 7 illustrates a block diagram of the needle-guidance system in accordance with some embodiments.
FIG. 8 illustrates a catheter-insertion device including a magnetizable needle in accordance with some embodiments.
FIG. 9 provides an exploded view of the catheter-insertion device of FIG. 5 in accordance with some embodiments.
FIG. 10 illustrates insertion of a needle into a blood vessel using an ultrasound probe of a needle-guidance system in accordance with some embodiments.
FIG. 11 illustrates an existing needle-guidance system along with needle packaging having a needle magnetizer and RFID-tag reader.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, there is an ongoing need to reduce operating costs for both medical facilities and patients alike. There is also an ongoing need to reduce landfill waste for the sake of the environment. Disclosed herein are needle-guidance systems, components, and methods that address the foregoing needs.

For example, a needle-guidance system is set forth below including, in some embodiments, a console, a needle magnetizer incorporated into the console, an RFID-tag reader incorporated into the console, and an ultrasound probe. Features of the console, the needle magnetizer, the RFID-tag reader, and the ultrasound probe will become more apparent to those of skill in the art in view of the accompanying drawings and following description, which describes the foregoing needle-guidance system, as well as other embodiments in greater detail.

### Needle-guidance systems

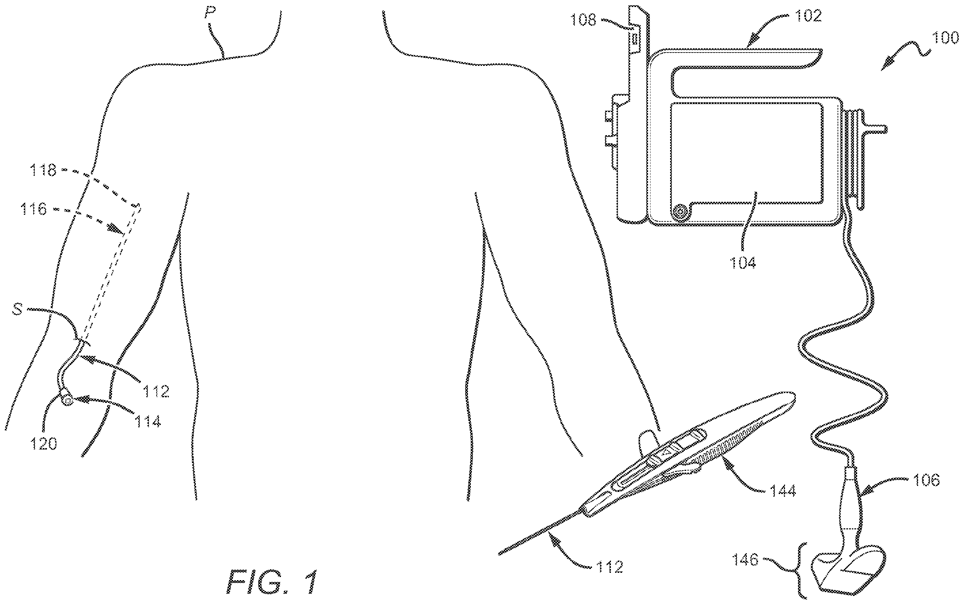

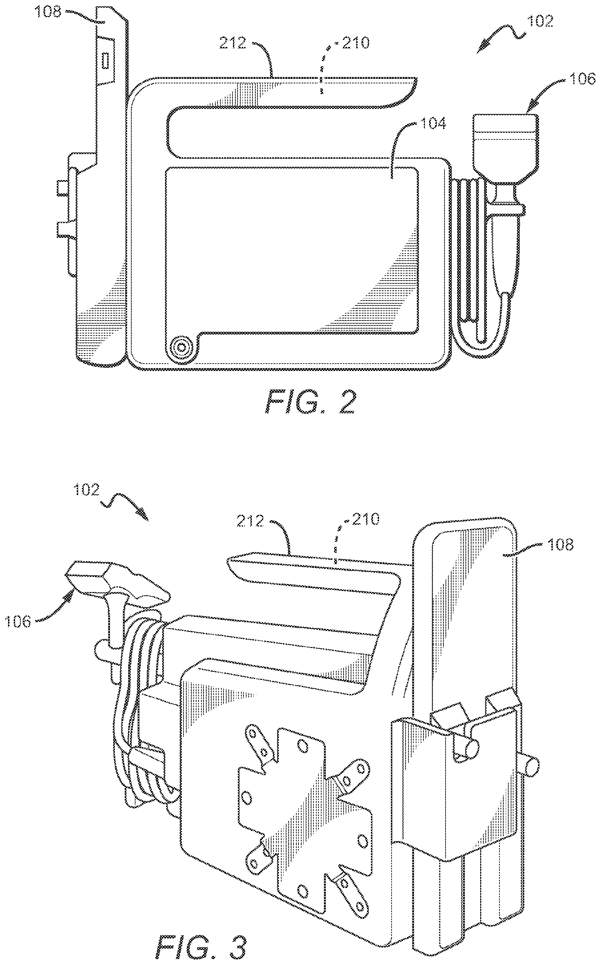

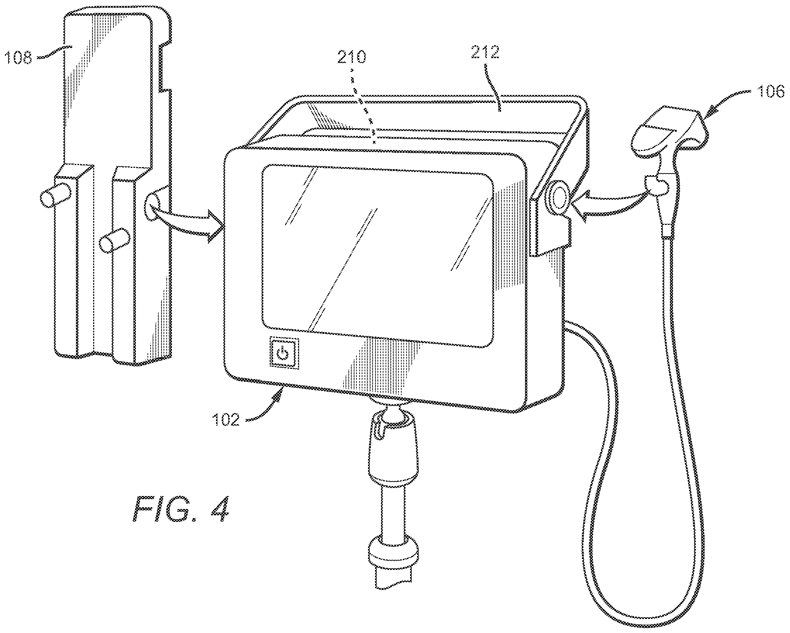

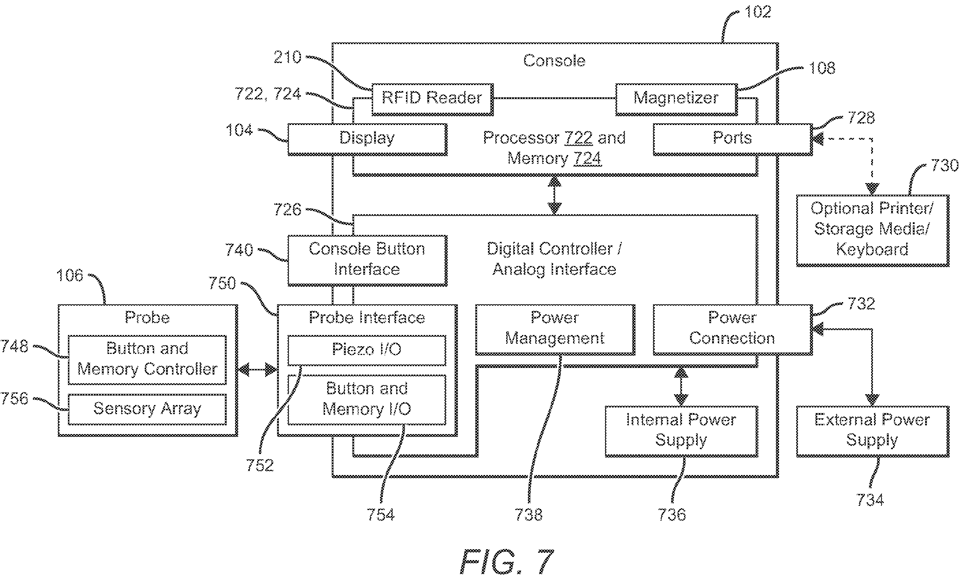

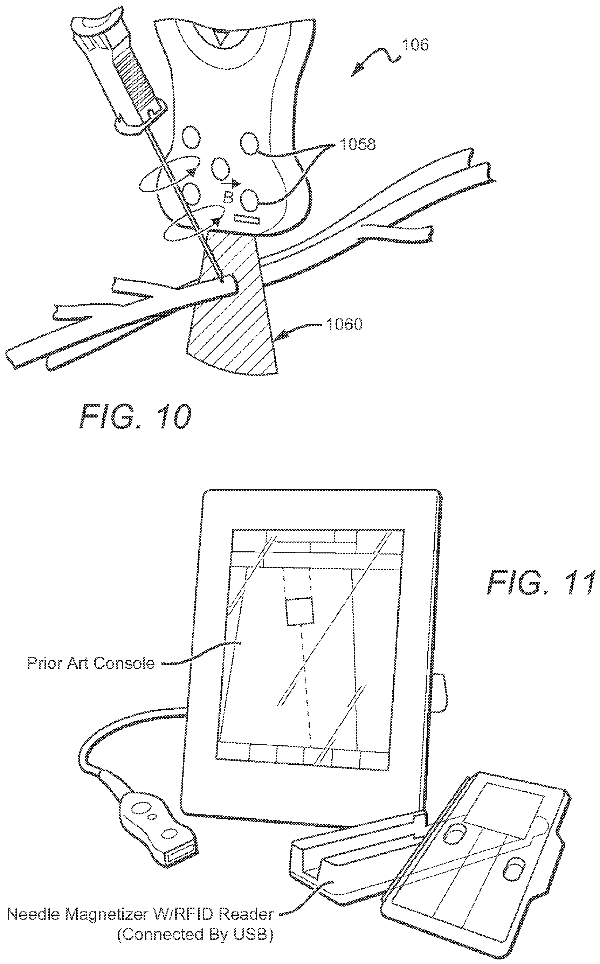

FIG. 1 illustrates a needle-guidance system 100 and a patient P in accordance with some embodiments. FIGS. 2 and 3 respectively illustrate a back and a front of a console 102 of the needle-guidance system 100 in accordance with some embodiments. FIGS. 4-6 illustrate the console 102 of the needle-guidance system 100 in accordance with some other embodiments thereof. FIG. 7 illustrates a block diagram of the needle-guidance system 100 in accordance with some embodiments. FIG. 10 illustrates an ultrasound probe 106 of the needle-guidance system 100 in accordance with some embodiments.

The needle-guidance system 100 is configured for locating and guiding a needle (e.g., the needle 808 of the catheter-insertion device 144) or another magnetizable medical component during ultrasound-based or other suitable procedures in order to access a subcutaneous target (e.g., blood vessel) of a patient. In some embodiments, the needle-guidance system 100 enables the position, orientation, and advancement of the needle to be superimposed in real-time atop an ultrasound image of the target, thus enabling a clinician to accurately guide the needle to the intended target. Furthermore, in some embodiments, the needle-guidance system 100 tracks a position of the needle in five degrees of motion: X, Y, and Z coordinate space, needle pitch, and needle yaw. Such tracking enables the needle to be guided and placed with relatively high accuracy.

As shown in FIGS. 1-7, the needle-guidance system 100 generally includes an ultrasound-imaging portion including the console 102, a display screen 104, and the ultrasound probe 106. In addition to the ultrasound-imaging portion of the needle-guidance system 100, the needle-guidance system 100 further includes a needle magnetizer 108 incorporated into the console 102, an RFID-tag reader 210 incorporated into the console 102, or both the needle magnetizer 108 and the RFID-tag reader 210 incorporated into the console 102.

The ultrasound-imaging portion of the needle-guidance system 100 is employed to image a target within a body of a patient such as a blood vessel prior to percutaneous insertion of a needle (e.g., the needle 808 of the catheter-insertion device 144) or another magnetizable medical component to access the target. *(See* FIG. 10.) In some embodiments, insertion of the needle is performed prior to or simultaneously with insertion of a catheter (e.g., the catheter 112 of the catheter-insertion device 144) into a blood vessel or another portion of a vasculature of the patient *P.* It should be appreciated that insertion of the needle into the body of the patient *P* can be performed for a variety of medical purposes other than or in addition to catheter insertion.

FIG. 1 shows the general relation of the needle-guidance system 100 and components thereof to the patient *P* during a procedure to place a catheter 112 into a vasculature of the patient *P* through a skin insertion site S. FIG. 1 shows that the catheter 112 generally includes a proximal portion 114 that remains exterior to the patient and a distal potion 116 that resides within the vasculature after placement is complete. The needle-guidance system 100 is employed to ultimately position a distal tip 118 of the catheter 112 in a desired position within the vasculature.

The proximal portion 114 of the catheter 112 further includes a Luer connector 120 configured to operably connect the catheter 112 with one or more other medical devices or systems. Placement of a needle into a vasculature of a patient such as the patient *P* at the insertion site *S* is typically performed prior to insertion of the catheter 112. It should be appreciated the needle-guidance system 100 has a variety of uses including placement of needles in preparation for inserting the catheter 112 or other medical components into a body of a patient such as X-ray or ultrasound markers, biopsy sheaths, ablation components, bladder scanning components, vena cava filters, etc.

The console 102 houses a variety of components of the needle-guidance system 100 and it is appreciated that the console 102 can take any of a variety of forms. A processor 722, including memory 724 such as random-access memory ("RAM") and non-volatile memory (e.g., electrically erasable programmable read-only memory ["EEPROM"]) is included in the console 102 for controlling system function and executing various algorithms during operation of the needle-guidance system 100. For example, the console 102 is configured to instantiate a needle-guidance process for guiding insertion of a needle into a target (e.g., blood vessel) of a patient. The needle-guidance process uses a combination of ultrasound-imaging data and magnetic-field data received by the console 102 for guiding the insertion of the needle into the target of the patient. A digital controller/analog interface 726 is also included with the console 102 and is in communication with both the processor 722 and other system components to govern interfacing between the ultrasound probe 106, the needle magnetizer 108, and the RFID-tag reader 210 and other system components.

The needle-guidance system 100 further includes ports 728 for connection with additional components such as optional components 730 including a printer, storage media, keyboard, etc. The ports 728 in some embodiments are universal serial bus ("USB") ports, though other port types or a combination of port types can be used for this and the other interfaces connections described herein. A power connection 732 is included with the console 102 to enable operable connection to an external power supply 734. An internal power supply 736 (e.g., a battery) can also be employed either with or exclusive of the external power supply 734. Power management circuitry 738 is included with the digital controller/analog interface 726 of the console 102 to regulate power use and distribution.

The display screen 104 is integrated into the console 102 and is used to display information to the clinician during the placement procedure such as an ultrasound image of the targeted internal body portion attained by the ultrasound probe 106. Indeed, the needle-guidance process of the needle-guidance system 100 graphically guides insertion of a needle into a target (e.g., a blood vessel) of a patient by way of the display screen 104. Notwithstanding the foregoing. the display screen 104 can alternatively be separate from the console 102. A console button interface 740 and any control buttons included on the ultrasound probe 106 can be used to immediately call up a desired mode to the display screen 104 by the clinician to assist in the placement procedure. In some embodiments, the display screen 104 is an LCD device.

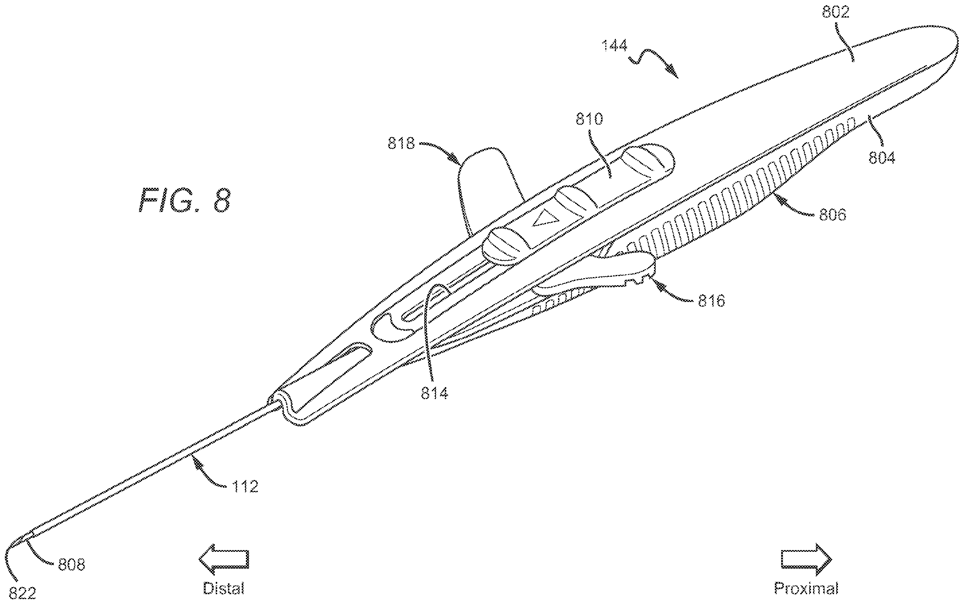

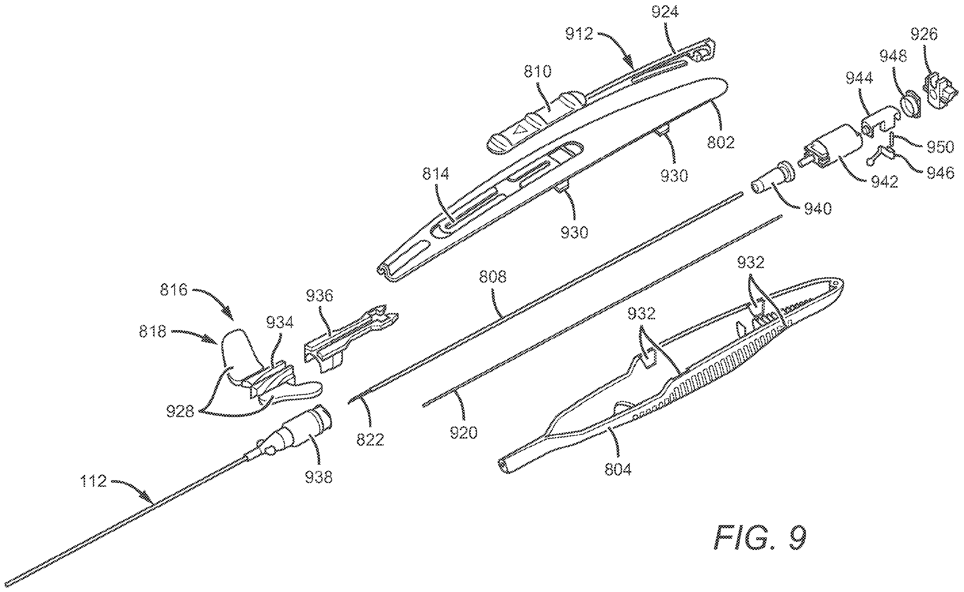

FIG. 1 further depicts a needle-based device, namely a catheter-insertion device 144 further depicted in FIGS. 8 and 9, used to gain initial access to the vasculature of the patient *P* via the insertion site *S* to deploy the catheter 112. As will be described in further detail below, the needle 808 of the catheter-insertion device 144 is configured to cooperate with the needle-guidance system 100 in enabling the needle-guidance system 100 to detect the position, orientation, and advancement of the needle 808 during an ultrasound-based placement procedure. It should be appreciated the needle 808 of the catheter-insertion device 144 is merely one example of a needle or medical device that can be magnetized and used with the needle-guidance system 100.

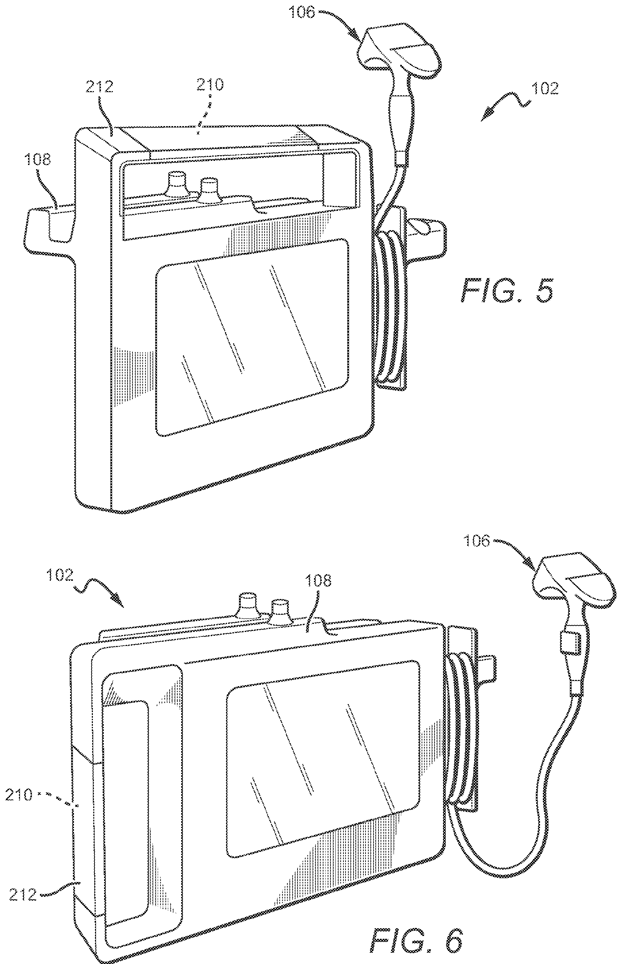

FIGS. 1-6 also show the needle magnetizer 108 incorporated into the console 102. The needle magnetizer 108 can be irremovably or removably coupled to a side, top, or back of the console 102. For example, FIGS. 1-4 illustrate the needle magnetizer 108 incorporated into a side of the console 102. Indeed, the needle magnetizer 108 is removably coupled to the side of the console 102 by a bracket in FIGS. 1-3 and a magnet in FIG. 4; however, the needle magnetizer 108 shares a housing with that of the console 102 and is, therefore, irremovably coupled to the top of the console 102 in FIGS. 5 and 6. The removably coupled needle magnetizer 108 is advantageous in that it can be removed as needed for cleaning, repairing, or replacing with another needle magnetizer like the needle magnetizer 108. That said, the irremovably coupled needle magnetizer 108 is advantageous in that it cannot be removed and misplaced.

The needle magnetizer 108 is configured to magnetize all or a portion of a needle such as the needle 808 of the catheter-insertion device 144 so as to enable the needle to be tracked during a placement procedure. The needle magnetizer 108 can include a single permanent magnet, a single electromagnet, a plurality of permanent magnets, a plurality of electromagnets, or a combination thereof within a body of the needle magnetizer 108 for magnetizing the needle. For example, the needle magnetizer 108 can include a single permanent magnet or a plurality of permanent magnets disposed within a body of the needle magnetizer 108 for magnetizing the needle. If a single permanent magnet, the permanent magnet can be a hollow cylindrical magnet disposed within the body of the needle magnetizer 108. If more than one permanent magnet, the permanent magnets can be disposed within the body of the needle magnetizer 108 in a multipole arrangement. Alternatively, the permanent magnets are annular magnets disposed within the body of the needle magnetizer 108 in a stacked arrangement.

FIGS. 2-6 also show the RFID-tag reader 210 incorporated into the console 102. The RFID-tag reader 210 can be incorporated into a side, top, or back of the console 102. For example, FIGS. 2-5 illustrate the RFID-tag reader 210 incorporated into the top of the console 102. Indeed, the RFID-tag reader 210 is incorporated into the top of the console 102 under a housing of the console in FIG. 4 and within a handle 212 on top of the console 102 in FIGS. 2, 3, and 5. In another example, FIG. 6 illustrates the RFID-tag reader 210 incorporated into the side of the console 102, specifically within the handle 212 on the side the console 102.

The RFID-tag reader 210 is configured to read at least passive RFID tags included with needles or other medical devices (e.g., the catheter-insertion device 144), which enables the needle-guidance system 100 to customize its operation to particular needle or medical-device parameters (e.g., type, size, etc.). For example, the needle-guidance process, once instantiated by the needle-guidance system 100, is configured to adjust needle-guidance parameters in accordance with electronically stored information read from an RFID tag for a needle. In order to read such an RFID tag, the RFID-tag reader is configured to emit interrogating radio waves into the RFID tag and read electronically stored information from the RFID tag.

The ultrasound probe 106 is employed in connection with ultrasound-based visualization of a target such as a blood vessel (*see* FIG. 10) in preparation for insertion of a needle into the target. Such visualization gives real-time ultrasound guidance and assists in reducing complications typically associated with such insertion, including inadvertent arterial puncture, hematoma, pneumothorax, etc. As described in more detail below, the ultrasound probe 106 is configured to provide to the console 102 electrical signals corresponding to both the ultrasound-imaging data and the magnetic-field data for the real-time ultrasound guidance.

The ultrasound probe 106 includes a head 146 that houses an array of piezoelectric transducers. The head 146 is configured for placement against a patient's skin proximate a prospective insertion site where the head 146 can produce ultrasonic pulses by way of the array of piezoelectric transducers, receive ultrasound echoes or reflected ultrasound signals after reflection of the ultrasonic pulses by the patient's body, and convert the reflected ultrasound signals from the patient into an ultrasound-imaging portion of the foregoing electrical signals to the console 102. In this way, a clinician can employ the ultrasound-imaging portion of the needle-guidance system 100 to determine a suitable insertion site and establish vascular access with a needle such as the needle 808 of the catheter-insertion device 144.

The ultrasound probe 106 can further include control buttons for controlling certain aspects of the needle-guidance system 100 during a procedure, thus eliminating the need for a clinician to reach out of a sterile field, which is established about the patient insertion site prior to establishment of the insertion site, to control the needle-guidance system 100.

FIG. 7 shows that the ultrasound probe 106 further includes a button-and-memory controller 748 for governing button and ultrasound probe operation. The button-and-memory controller 748 can include non-volatile memory (e.g., EEPROM). The button-and-memory controller 748 is in operable communication with a probe interface 750 of the console 102, which includes a piezo input/output component 752 for interfacing with the probe piezoelectric array and a button-and-memory input/output component 754 for interfacing with the button-and-memory controller 748.

Also as seen in FIGS. 7 and 10, the ultrasound probe 106 includes a sensor array 756 for detecting the position, orientation, and movement of a needle during ultrasound imaging procedures. The sensor array 756 includes a number of magnetic sensors 1058 embedded within or included on a housing of the ultrasound probe 106. The magnetic sensors 1058 are configured to detect a magnetic field or magnetic signals associated with a needle when the needle is magnetized and in proximity to the sensor array 756, as well as convert the magnetic signals from the magnetized needle into a magnetic-field portion of the foregoing electrical signals to the console 102. (*See* the magnetic field *B* of the needle in FIG. 10.) Thus, the sensor array 756 enables the needle-guidance system 100 to track a magnetized needle or the like.

Though configured here as magnetic sensors, it is appreciated that the magnetic sensors 1058 can be sensors of other types and configurations. Also, though they are described herein as included with the ultrasound probe 106, the magnetic sensors 1058 of the sensor array 756 can be included in a component separate from the ultrasound probe 106, such as a separate handheld device. In some embodiments, the magnetic sensors 1058 are disposed in an annular configuration about the head 146 of the ultrasound probe 106, though it is appreciated that the magnetic sensors 1058 can be arranged in other configurations, such as in an arched, planar, or semi-circular arrangement.

Each magnetic sensor of the magnetic sensors 1058 includes three orthogonal sensor coils for enabling detection of a magnetic field in three spatial dimensions. Such 3-dimensional ("3-D") magnetic sensors can be purchased, for example, from Honeywell Sensing and Control of Morristown, NJ. Further, the magnetic sensors 1058 are configured as Halleffect sensors, though other types of magnetic sensors could be employed. Further, instead of 3-D sensors, a plurality of 1-dimensional ("1-D") magnetic sensors can be included and arranged as desired to achieve 1-, 2-, or 3-D detection capability.

Five magnetic sensors 1058 are included in the sensor array 756 so as to enable detection of a needle three spatial dimensions (i.e., X, Y, Z coordinate space), as well as the pitch and yaw orientation of the needle itself. Note that in some embodiments, orthogonal sensing components of two or more of the magnetic sensors 1058 enable the pitch and yaw attitude of the needle to be determined. In other embodiments, fewer than five or more than five magnetic sensors of the magnetic sensors 1058 can be employed in the sensor array 756. More generally, it is appreciated that the number, size, type, and placement of the magnetic sensors 1058 of the sensor array 756 can vary from what is explicitly shown here.

It is appreciated that a needle of a magnetizable material enables the needle to be magnetized by the needle magnetizer 108 and later be tracked by the needle-guidance system 100 when the needle is percutaneously inserted into a body of a patient (e.g., the body of the patient *P*) during a procedure to insert the needle or an associated medical device (e.g., the catheter 112 of the catheter-insertion device 144) into the body of the patient *P.* In some embodiments, the needle is composed of a stainless steel such as SS 304 stainless steel; however, other suitable needle materials that are capable of being magnetized can be employed. In some embodiments, the needle material is ferromagnetic. In other embodiments, the needle is paramagnetic. So configured, the needle can produce a magnetic field or magnetic signals detectable by the sensor array 756 of the ultrasound probe 106 so as to enable the location, orientation, and movement of the needle to be tracked by the needle-guidance system 100.

During operation of the needle-guidance system 100, the head 146 of the ultrasound probe 106 is placed against skin of a patient and produces an ultrasound beam 1060 so as to ultrasonically image a portion of a target such as a blood vessel beneath a surface of the skin of the patient. *(See* FIG. 10.) The ultrasonic image of the blood vessel can be depicted on the display screen 104 of the needle-guidance system 100.

The needle-guidance system 100 is configured to detect the position, orientation, and movement of a needle such as the needle 808 of the catheter-insertion device 144. In particular, the sensor array 756 of the ultrasound probe 106 is configured to detect a magnetic field of the needle after magnetization thereof. Each magnetic sensor of the magnetic sensors 1058 in the sensor array 756 is configured to spatially detect the magnetized needle in 3-dimensional space. *(See* FIG. 10.) Thus, during operation of the needle-guidance system 100, magnetic field strength data of the needle's magnetic field sensed by each magnetic sensor of the magnetic sensors 1058 is forwarded to a processor such as the processor 722 of the console 102, which computes in real-time the position or orientation of the needle for graphical display on the display screen 104.

The position of the entire length of a needle in X, Y, and Z coordinate space with respect to the sensor array 756 can be determined by the needle-guidance system 100 using the magnetic field strength data sensed by the magnetic sensors 1058. Moreover, the pitch and yaw of the needle can also be determined. Suitable circuitry of the ultrasound probe 106, the console 102, or other components of the needle-guidance system 100 can provide the calculations necessary for such position or orientation. In some embodiments, the needle can be tracked using the teachings of one or more of the following U.S. Patent Nos.: 5,775,322; 5,879,297; 6,129,668; 6,216,028; and 6,263,230.

The position and orientation information determined by the needle-guidance system 100, together with an entire length of a needle, as known by or input into the needle-guidance system 100 such as reading an RFID tag associated with the needle, enables the needle-guidance system 100 to accurately determine the location and orientation of the entire length of the needle, including the distal tip of the needle, with respect to the sensor array 756. This, in turn, enables the needle-guidance system 100 to superimpose an image of the needle on an image produced by the ultrasound beam 1060 of the ultrasound probe 106 on the display screen 104. For example, the ultrasound image depicted on the display screen 104 can include depiction of the surface of the skin of the patient *P* and a subcutaneous blood vessel thereunder to be accessed by the needle, as well as a depiction of the needle as detected by the needle-guidance system 100 and its position with respect to the vessel. The ultrasound image corresponds to an image acquired by the ultrasound beam of the ultrasound probe 106. In other embodiments, it is appreciated that only a portion of the entire length of the needle is magnetized and thus tracked by the needle-guidance system 100.

Note that further details regarding structure and operation of the needle-guidance system 100 can be found in U.S. Patent No. 9,756,766, titled "Apparatus for Use with Needle Insertion Guidance System,".

A needle such as the needle 808 of the catheter-insertion device 144 can be magnetized so as to be trackable by the needle-guidance system 100 when the needle is inserted into the body of the patient *P*. Such magnetic-based tracking of the needle assists the clinician in placing a distal tip of the needle in a desired location, such as in the lumen of a blood vessel, by superimposing a simulated needle image representing the real-time position and orientation of the needle over an ultrasound image of the internal area of the patient body being accessed by the needle.

A needle can be included as part of a medical device such as the catheter-insertion device 144, though many other implementations with other types of medical devices are contemplated. Indeed, even a stand-alone needle magnetized with the needle magnetizer 108 can be guided by the needle-guidance system 100.

### Catheter-insertion device

FIGS. 8 and 9 depict various details of the catheter-insertion device 144 for assistance in inserting the catheter 112 into a body of a patient, which also serves as an example of a medical device including a needle that can be magnetized and used with the needle-guidance system 100 according to some embodiments. As shown in FIG. 8, the catheter-insertion device 144 includes top and bottom housing portions 802 and 804 of a housing 806, from which extends the catheter 112 disposed over a needle 808. Also shown is a finger pad 810 of a guidewire advancement assembly 912 slidably disposed in a slot 814 defined in the top housing portion 802, and a portion of a handle assembly 816 of a catheter advancement assembly 818.

FIGS. 8 and 9 also show that the finger pad 810 as part of the guidewire advancement assembly 912 can be slid by one or more fingers of the user distally along the slot 814 in order to enable selective advancement of a guidewire 920 (initially disposed within a lumen of the needle 808) out past a distal end or tip 822 of the needle 808. A proximal end of the guidewire 920 is attached to an interior portion of the top housing portion 802 such that a single unit of sliding advancement of the finger pad 810 in a distal direction results in two units of guidewire advancement in the distal direction. This is made possible by looping the guidewire 920 from its attachment point on the top housing portion 802 through guide surfaces included on a guidewire lever 924 before extending into the lumen of the needle 808. Note that the guidewire lever 924 and the finger pad 810 of the guidewire advancement assembly 912 are integrally formed with one another, though they can be separately formed in other embodiments. Note also that the guidewire 920 can be attached to other external or internal portions of the catheter-insertion device 144, including the bottom housing portion 804, a needle hub 926, etc.

FIGS. 8 and 9 further show that the catheter advancement assembly 818 for selectively advancing the catheter 112 in a distal direction out from the housing 806 of the catheter-insertion device 144 includes the handle assembly 816, which in turn includes among other components two wings 928 that are grasped by the fingers of the user when the catheter 112 is to be advanced. The wings 928 distally advance via a gap defined between the top and bottom housing portions 802 and 804.

The top and bottom housing portions 802 and 804 are mated together via the engagement of four tabs 930 of the top housing portion 802 with four corresponding recesses 932 located on the bottom housing portion 804.

The exploded view of the catheter-insertion device 144 in FIG. 9 shows that the handle assembly 816 includes a head portion 934 from which extend the wings 928 and a tail portion 936. Both the head portion 934 and the tail portion 936 are removably attached to a catheter hub 938 including the Luer connector 120. Internal components of the catheter-insertion device 144 are disposed within the housing 806. Each component of the internal components is passed through by the needle 808, the internal components including a valve 940, a safety housing 942, a carriage 944, a needle safety component 946, and a cap 948 of the safety housing 942. An O-ring 950 included with the needle safety component 946 is also shown, as is the needle hub 926, which is secured to a proximal end of the needle 808 and mounted to the housing 806 to secure the needle 808 in place within the catheter-insertion device 144. Note in FIG. 9 that the slot 814 in which the finger pad 810 of the guidewire advancement assembly 912 is disposed includes a relatively wide portion to enable the guidewire lever 924 to be inserted therethrough in order to couple the guidewire advancement assembly 912 to the housing 806.

The catheter-insertion device 144 is used by a clinician to insert the catheter 112 into a venous system (or other location) of a patient so as to enable fluids, medicaments, etc. to be infused into or removed from a patient. Though depicted here as a midline catheter, the catheter 112 can include any catheter of a variety of lengths, including relatively shorter peripheral catheters, peripherally inserted central catheters ("PICCS"), central venous catheters ("CVCs"), etc. Also, other elongate medical devices can be employed including solid and hollow needles and cannulas, blood-draw needles, biopsy needles, introducer needles, guidewires, stylets, tissue-penetrating medical components, etc. Further details regarding the catheter-insertion device 144 and its operation can be found in U.S. Patent No. 9,950,139, titled "Catheter Placement Device Including Guidewire and Catheter Control Elements,".

### Methods

A method of the needle-guidance system 100 includes an instantiating step of instantiating in the memory 724 (e.g., the RAM) of the console 102 the needle-guidance process for guiding insertion of a needle into a blood vessel of a patient using a combination of ultrasound-imaging data and magnetic-field data.

The method can further include a magnetizing step of magnetizing the needle in accordance with a first preparatory step for the insertion of the needle into the blood vessel of the patient. The magnetizing step includes magnetizing the needle with the needle magnetizer 108 incorporated into the console 102 when the needle is inserted into the needle magnetizer 108. The magnetizing step produces a magnetized needle.

The method can further include a reading step of reading electronically stored information for the needle in accordance with a second preparatory step for the insertion of the needle into the blood vessel of the patient. The reading step includes reading into the memory 724 the electronically stored information from an RFID tag for the needle. The reading step is effected with interrogating radio waves emitted by the RFID-tag reader 210 incorporated into the console 102. After the reading step, the method can further include an adjusting step of adjusting needle-guidance parameters in the needle-guidance process in accordance with the electronically stored information read from the RFID tag.

With respect to the ultrasound probe 106, the method further includes an ultrasound-signal converting step of converting patient-reflected ultrasound signals into an ultrasound-imaging portion of the electrical signals with the array of piezoelectric transducers of the ultrasound probe 106. The method further includes a magnetic-signal converting step of converting magnetic signals into a magnetic-field portion of the electrical signals with the sensor array 756 of the magnetic sensors 1058 of the ultrasound probe 106.

The method further includes a loading step of loading the ultrasound-imaging data and the magnetic-field data in the memory 724. The ultrasound-imaging data and magnetic-field data correspond to electrical signals received from the ultrasound probe 106. Following the loading step, the method further includes a processing step of processing the ultrasound-imaging data and the magnetic-field data with the processor 722 of the console 102 for a guiding step of the method for graphically guiding the insertion of the magnetized needle into the blood vessel of the patient on the display screen 104 of the console 102.

A method associated with the needle-guidance system 100 includes an obtaining step of obtaining a needle.

In accordance with the first preparatory step set forth above, the method can further include an inserting step of inserting the needle into the needle magnetizer 108 incorporated into the console 102 to produce a magnetized needle.

In accordance with the second preparatory step set forth above, the method can further include a causing step of causing the RFID-tag reader 210 incorporated into the console 102 to read electronically stored information from an RFID tag for the needle before or after producing the magnetized needle. The causing step can include merely bringing the RFID tag into range of the RFID-tag reader 210. The causing step further causes the needle-guidance process to adjust needle-guidance parameters for the insertion of the magnetized needle into a blood vessel of a patient in accordance with the electronically stored information read from the RFID tag.

As shown in FIG. 10, the method further includes an imaging step of imaging the blood vessel of the patient with the ultrasound probe 106 to produce ultrasound-imaging data. FIG. 10 also shows the method further includes an orienting step of orienting the magnetized needle for insertion into the blood vessel of the patient to produce magnetic-field data while imaging the blood vessel with the ultrasound probe 106.

The method can further include an inserting step of inserting the magnetized needle into the blood vessel of the patient in accordance with graphical guidance on the display screen 104 of the console 102 for insertion of the magnetized needle into the blood vessel of the patient. The guidance is provided by a needle-guidance process instantiated by the console 102 upon processing a combination of the ultrasound-imaging data and the magnetic-field data.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A needle-guidance system (100), comprising:
a console (102) including memory (724) and a processor (722) configured to instantiate a needle-guidance process for guiding insertion of a needle into a blood vessel of a patient using a combination of ultrasound-imaging data and magnetic-field data received by the console (102);
a needle magnetizer (108) incorporated into the console (102) configured to magnetize the needle; and
an ultrasound probe (106) configured to provide to the console (102) electrical signals corresponding to both the ultrasound-imaging data and the magnetic-field data, the ultrasound probe (106) including:
an array of piezoelectric transducers configured to convert reflected ultrasound signals from the patient into an ultrasound-imaging portion of the electrical signals; and
an array of magnetic sensors (1058) configured to convert magnetic signals from the magnetized needle into a magnetic-field portion of the electrical signals.

2. The needle-guidance system of claim 1, wherein the needle magnetizer (108) is removably or irremovably coupled to a side, top, or back of the console (102).

3. The needle-guidance system of claim 1 or 2, wherein the needle magnetizer (108) includes a single permanent magnet or a plurality of permanent magnets disposed within a body of the needle magnetizer (108) for magnetizing the needle.

4. The needle-guidance system of claim 3, wherein the permanent magnets are disposed within the body of the needle magnetizer (108) in a multipole arrangement.

5. The needle-guidance system of claim 3, wherein the permanent magnets are annular magnets disposed within the body of the needle magnetizer (108) in a stacked arrangement.

6. The needle-guidance system of claim 3, wherein the permanent magnet is hollow cylindrical magnet disposed within the body of the needle magnetizer (108).

7. The needle-guidance system of claim 1 or 2, wherein the needle magnetizer (108) includes a single electromagnet or a plurality of electromagnets disposed within a body of the needle magnetizer (108) for magnetizing the needle.

8. The needle-guidance system of any claim of claims 1-7, further comprising a radiofrequency identification ("RFTD")-tag reader (210) incorporated into the console (102) configured to emit interrogating radio waves into a passive RFID tag for the needle and read electronically stored information from the RFID tag.

9. The needle-guidance system of claim 8, wherein the needle-guidance process is configured to adjust needle-guidance parameters in accordance with the electronically stored information read from the RFID tag.

10. The needle-guidance system of any claim of claims 1-9, further comprising a display screen (104) configured for graphically guiding the insertion of the needle into the blood vessel of the patient.

11. A console (102) for a needle-guidance system, comprising:
a processor (722) and memory (724) configured to instantiate a needle-guidance process for guiding insertion of a needle into a blood vessel of a patient using a combination of ultrasound-imaging data and magnetic-field data received by the console (102);
a needle magnetizer (108) incorporated into the console (102) configured to magnetize the needle;
a probe interface (750) configured to provide to the console (102) electrical signals from an ultrasound probe (106), the electrical signals corresponding to both the ultrasound-imaging data and the magnetic-field data; and
a display screen (104) configured for graphically guiding the insertion of the needle into the blood vessel of the patient.

12. The console (102) of claim 11, wherein the needle magnetizer (108) is removably or irremovably coupled to a side, top, or back of the console (102).

13. The console (102) of claim 11 or 12, wherein the needle magnetizer (108) includes a single permanent magnet or a plurality of permanent magnets disposed within a body of the needle magnetizer (108) for magnetizing the needle.

14. The console (102) of claim 11 or 12, wherein the needle magnetizer (108) includes a single electromagnet or a plurality of electromagnets disposed within a body of the needle magnetizer (108) for magnetizing the needle.

15. The console (102) of any claim of claims 11-14, further comprising a radiofrequency identification ("RFTD")-tag reader (210) incorporated into the console (102) configured to emit interrogating radio waves into a passive RFID tag for the needle and read electronically stored information from the RFID tag, preferably wherein the needle-guidance process is configured to adjust needle-guidance parameters in accordance with the electronically stored information read from the RFID tag.

## Patentansprüche

1. Nadelführungssystem (100), umfassend:
eine Konsole (102), die einen Speicher (724) und einen Prozessor (722) einschließt, die dafür konfiguriert sind, einen Nadelführungsprozess zum Führen der Einführung einer Nadel in ein Blutgefäß eines Patienten unter Verwendung einer Kombination von Ultraschall-Bildgebungsdaten und Magnetfelddaten zu realisieren, die durch die Konsole (102) empfangen werden;
einen Nadelmagnetisierer (108), der in die Konsole (102) eingegliedert ist und dafür konfiguriert ist, die Nadel zu magnetisieren; und
eine Ultraschallsonde (106), die dafür konfiguriert ist, elektrische Signale an die Konsole (102) bereitzustellen, die sowohl den Ultraschall-Bildgebungsdaten als auch den Magnetfelddaten entsprechen, wobei die Ultraschallsonde (106) einschließt:
ein Array von piezoelektrischen Wandlern, die dafür konfiguriert sind, reflektierte Ultraschallsignale von dem Patienten in einen Ultraschall-Bildgebungsteil der elektrischen Signale umzuwandeln; und
ein Array von magnetischen Sensoren (1058) die dafür konfiguriert sind, magnetische Signale von der magnetisierten Nadel in einen Magnetfeldteil der elektrischen Signale umzuwandeln.

2. Nadelführungssystem nach Anspruch 1, wobei der Nadelmagnetisierer (108) entfernbar oder nicht entfernbar mit einer Seite, Oberseite oder Rückseite der Konsole (102) verbunden ist.

3. Nadelführungssystem nach Anspruch 1 oder 2, wobei der Nadelmagnetisierer (108) einen einzelnen Permanentmagneten oder eine Vielzahl von Permanentmagneten einschließt, die in einem Körper des Nadelmagnetisierers (108) zum Magnetisieren der Nadel angeordnet sind.

4. Nadelführungssystem nach Anspruch 3, wobei die Permanentmagneten in dem Körper des Nadelmagnetisierers (108) in einer mehrpoligen Anordnung angeordnet sind.

5. Nadelführungssystem nach Anspruch 3, wobei es sich bei den Permanentmagneten um Ringmagneten handelt, die in dem Körper des Nadelmagnetisierers (108) in einer gestapelten Anordnung angeordnet sind.

6. Nadelführungssystem nach Anspruch 3, wobei der Permanentmagnet ein hohler zylindrischer Magnet ist, der in dem Körper des Nadelmagnetisierers (108) angeordnet ist.

7. Nadelführungssystem nach Anspruch 1 oder 2, wobei der Nadelmagnetisierer (108) einen einzelnen Elektromagneten oder eine Vielzahl von Elektromagneten einschließt, die in einem Körper des Nadelmagnetisierers (108) zum Magnetisieren der Nadel angeordnet sind.

8. Nadelführungssystem nach einem der Ansprüche 1-7, weiter umfassend eine Leseeinrichtung (210) für Radiofrequenz-Identifikations-Tags ("RFID"-Tags), die in die Konsole (102) eingegliedert ist und dafür konfiguriert ist, Funkwellen zur Abfrage in ein passives RFID-Tag für die Nadel auszusenden und elektronisch gespeicherte Informationen aus dem RFID-Tag auszulesen.

9. Nadelführungssystem nach Anspruch 8, wobei der Nadelführungsprozess dafür konfiguriert ist, Nadelführungsparameter in Übereinstimmung mit den elektronisch gespeicherten Informationen einzustellen, die aus dem RFID-Tag ausgelesen werden.

10. Nadelführungssystem nach einem der Ansprüche 1-9, weiter umfassend einen Anzeigebildschirm (104), der dafür konfiguriert ist, die Einführung der Nadel in das Blutgefäß des Patienten grafisch zu führen.

11. Konsole (102) für ein Nadelführungssystem, umfassend:
einen Prozessor (722) und einen Speicher (724), die dafür konfiguriert sind, einen Nadelführungsprozess zum Führen der Einführung einer Nadel in ein Blutgefäß eines Patienten unter Verwendung einer Kombination von Ultraschall-Bildgebungsdaten und Magnetfelddaten zu realisieren, die durch die Konsole (102) empfangen werden;
einen Nadelmagnetisierer (108), der in die Konsole (102) eingegliedert ist und dafür konfiguriert ist, die Nadel zu magnetisieren;
eine Sondenschnittstelle (750), die dafür konfiguriert ist, elektrische Signale von einer Ultraschallsonde (106) an die Konsole (102) bereitzustellen, wobei die elektrischen Signale sowohl den Ultraschall-Bildgebungsdaten als auch den Magnetfelddaten entsprechen; und
einen Anzeigebildschirm (104), der dafür konfiguriert ist, die Einführung der Nadel in das Blutgefäß des Patienten grafisch zu führen.

12. Konsole (102) nach Anspruch 11, wobei der Nadelmagnetisierer (108) entfernbar oder nicht entfernbar mit einer Seite, Oberseite oder Rückseite der Konsole (102) verbunden ist.

13. Konsole (102) nach Anspruch 11 oder 12, wobei der Nadelmagnetisierer (108) einen einzelnen Permanentmagneten oder eine Vielzahl von Permanentmagneten einschließt, die in einem Körper des Nadelmagnetisierers (108) zum Magnetisieren der Nadel angeordnet sind.

14. Konsole (102) nach Anspruch 11 oder 12, wobei der Nadelmagnetisierer (108) einen einzelnen Elektromagneten oder eine Vielzahl von Elektromagneten einschließt, die in einem Körper des Nadelmagnetisierers (108) zum Magnetisieren der Nadel angeordnet sind.

15. Konsole (102) nach einem der Ansprüche 11-14, weiter umfassend eine Leseeinrichtung (210) für Radiofrequenz-Identifikations-Tags ("RFID"-Tags), die in die Konsole (102) eingegliedert ist und dafür konfiguriert ist, Funkwellen zur Abfrage in ein passives RFID-Tag für die Nadel auszusenden und elektronisch gespeicherte Informationen aus dem RFID-Tag auszulesen, vorzugsweise wobei der Nadelführungsprozess dafür konfiguriert ist, Nadelführungsparameter in Übereinstimmung mit den elektronisch gespeicherten Informationen einzustellen, die aus dem RFID-Tag ausgelesen werden.

## Revendications

1. Système (100) de guidage d'aiguille, comprenant :
une console (102) incluant une mémoire (724) et un processeur (722) configurés pour instancier un processus de guidage d'aiguille pour guider l'insertion d'une aiguille dans un vaisseau sanguin d'un patient en utilisant une combinaison de données d'imagerie par ultrasons et de données de champ magnétique reçues par la console (102) ;
un magnétiseur (108) d'aiguille incorporé dans la console (102) conçu pour magnétiser l'aiguille ; et
une sonde (106) à ultrasons conçue pour fournir à la console (102) des signaux électriques correspondant à la fois aux données d'imagerie par ultrasons et aux données de champ magnétique, la sonde (106) à ultrasons incluant :
un réseau de transducteurs piézoélectriques conçus pour convertir des signaux ultrasonores réfléchis par le patient en une portion d'imagerie par ultrasons des signaux électriques ; et
un réseau de capteurs (1058) magnétiques conçus pour convertir des signaux magnétiques provenant de l'aiguille magnétisée en une portion de champ magnétique des signaux électriques.

2. Système de guidage d'aiguille selon la revendication 1, dans lequel le magnétiseur (108) d'aiguille est couplé de manière amovible ou inamovible à un côté, sommet ou dos de la console (102).

3. Système de guidage d'aiguille selon la revendication 1 ou la revendication 2, dans lequel le magnétiseur (108) d'aiguille inclut un seul aimant permanent ou une pluralité d'aimants permanents disposés à l'intérieur d'un corps du magnétiseur (108) d'aiguille pour magnétiser l'aiguille.

4. Système de guidage d'aiguille selon la revendication 3, dans lequel les aimants permanents sont disposés à l'intérieur du corps du magnétiseur (108) d'aiguille dans un agencement à plusieurs pôles.

5. Système de guidage d'aiguille selon la revendication 3, dans lequel les aimants permanents sont des aimants annulaires disposés à l'intérieur du corps du magnétiseur (108) d'aiguille dans un agencement empilé.

6. Système de guidage d'aiguille selon la revendication 3, dans lequel l'aimant permanent est un aimant cylindrique creux disposé à l'intérieur du corps du magnétiseur (108) d'aiguille.

7. Système de guidage d'aiguille selon la revendication 1 ou la revendication 2, dans lequel le magnétiseur (108) d'aiguille inclut un seul électroaimant ou une pluralité d'électroaimants disposés à l'intérieur d'un corps du magnétiseur (108) d'aiguille pour magnétiser l'aiguille.

8. Système de guidage d'aiguille selon une quelconque revendication des revendications 1 à 7, comprenant en outre un lecteur (210) d'étiquette d'identification radiofréquence (« RFTD ») incorporé dans la console (102) conçu pour émettre des ondes radio d'interrogation dans une étiquette RFID passive de l'aiguille et lire des informations stockées par voie électronique à partir de l'étiquette RFID.

9. Système de guidage d'aiguille selon la revendication 8, dans laquelle le processus de guidage d'aiguille est conçu pour ajuster des paramètres de guidage d'aiguille conformément aux informations stockées par voie électronique provenant de l'étiquette RFID.

10. Système de guidage d'aiguille selon une quelconque revendication des revendications 1 à 9, comprenant en outre un écran (104) d'affichage conçu pour guider graphiquement l'insertion de l'aiguille dans le vaisseau sanguin du patient.

11. Console (102) pour un système de guidage d'aiguille, comprenant :
un processeur (722) et une mémoire (724) configurés pour instancier un processus de guidage d'aiguille pour guider l'insertion d'une aiguille dans un vaisseau sanguin d'un patient en utilisant une combinaison de données d'imagerie par ultrasons et de données de champ magnétique reçues par la console (102) ;
un magnétiseur (108) d'aiguille incorporé dans la console (102) conçu pour magnétiser l'aiguille ;
une interface (750) de sonde configurée pour fournir à la console (102) des signaux électriques provenant d'une sonde (106) à ultrasons, les signaux électriques correspondant à la fois aux données d'imagerie par ultrasons et aux données de champ magnétique ; et
un écran (104) d'affichage conçu pour guider graphiquement l'insertion de l'aiguille dans le vaisseau sanguin du patient.

12. Console (102) selon la revendication 11, dans lequel le magnétiseur (108) d'aiguille est couplé de manière amovible ou inamovible à un côté, sommet ou dos de la console (102).

13. Console (102) selon la revendication 11 ou la revendication 12, dans lequel le magnétiseur (108) d'aiguille inclut un seul aimant permanent ou une pluralité d'aimants permanents disposés à l'intérieur d'un corps du magnétiseur (108) d'aiguille pour magnétiser l'aiguille.

14. Console (102) selon la revendication 11 ou la revendication 12, dans lequel le magnétiseur (108) d'aiguille inclut un seul électroaimant ou une pluralité d'électroaimants disposés à l'intérieur d'un corps du magnétiseur (108) d'aiguille pour magnétiser l'aiguille.

15. Console (102) selon une quelconque revendication des revendications 11 à 14, comprenant en outre un lecteur (210) d'étiquette d'identification radiofréquence (« RFTD ») incorporé dans la console (102) conçu pour émettre des ondes radio d'interrogation dans une étiquette RFID passive pour l'aiguille et lire des informations stockées par voie électronique à partir de l'étiquette RFID, de préférence dans laquelle le processus de guidage d'aiguille est conçu pour ajuster des paramètres de guidage d'aiguille conformément aux informations stockées par voie électronique provenant de l'étiquette RFID.
